# EUROPÄISCHE PATENTANMELDUNG

(11) **EP 2 548 562 A1**
(43) Veröffentlichungstag der Anmeldung: **23.01.2013**
(21) Anmeldenummer: 11174348.0
(22) Anmeldetag: 18.07.2011
(51) Int. Cl.: A61K 33/36, A61P 13/12

(54) **Kombinationstherapie mit Eisen-basierenden Phosphatadsorbern**

(71) Anmelder: SeBo GmbH, 69117 Heidelberg (DE)
(72) Erfinder: Seidel, Dietrich, 83340 Feldafing (DE)
(74) Vertreter: Weiss, Wolfgang

(57) **Zusammenfassung**

Die Erfindung betrifft einen Eisen-basierenden Phosphatadsorber zur oralen Verabreichung für medizinische Zwecke in Kombination mit einer Calcium-Supplementation, z.B. durch Verabreichung eines physiologisch verträglichen Calciumsalzes und/oder calciumreicher Nahrungsmittel, und/oder in Kombination mit einem physiologisch verträglichen Anionenaustauscher.

## Beschreibung

Die Erfindung betrifft einen Eisen-basierenden Phosphatadsorber zur oralen Verabreichung für medizinische Zwecke in Kombination mit einer Calcium-Supplementation, z.B. durch Verabreichung eines physiologisch verträglichen Calciumsalzes und/oder calciumreicher Nahrungsmittel, und/oder in Kombination mit einem physiologisch verträglichen Anionenaustauscher.

DE 42 39 442.2 beschreibt die Verwendung eines Eisen-basierenden Phosphatadsorbers zur Behandlung der Hyperphosphatämie. Seither sind solche Produkte mehrfach in Untersuchungen an menschlichen Patienten eingesetzt worden (siehe z.B. Hergesell und Ritz, Nephrol. Dial. Transplant. 14 (1999), 863-867; Block et al., Kidney International Advance Online Publication, 10. März 2010, DOI:10.1038/Kl.2021.23; Geisser und Philipp, Clin. Nephrol., 74 (2010), 4-11).

WO 2010/100112 beschreibt eine Zusammensetzung, enthaltend eine Mischung von Calcium-, Magnesium- und Eisensalzen zur Verwendung als Phosphatadsorber, insbesondere zur Verwendung bei der Behandlung von Hyperphosphatämie, bei der Behandlung von chronischer Nierendefizienz sowie zur Behandlung von Hämodialysepatienten.

Obwohl nach bisherigen Ergebnissen die Verabreichung von Eisen-basierenden Phosphatadsorbern eine gute klinische Wirkung zeigt, wurde bei manchen Patienten eine Unverträglichkeit festgestellt, die sich in milder bis moderater Diarrhoe äußert.

Eine Aufgabe der vorliegenden Erfindung bestand darin, die Verträglichkeit von Eisen-basierenden Phosphatadsorbern zu verbessern und insbesondere bei Verabreichung auftretenden Unverträglichkeiten zu vermeiden.

Ein Aspekt der vorliegenden Erfindung beruht auf der Erkenntnis, dass die Verträglichkeit eines oral verabreichten Eisen-basierenden Phosphatadsorbers durch eine Calcium-Supplementation verbessert wird. Es wird angenommen, dass die Supplementation den, durch Adsorption des in der Nahrung vorhandenen Calciums an den Phosphatadsorber verursachten Calcium-Mangel im Magen-Darm-Kanal ausgleicht, wodurch die Verträglichkeit des Phosphatadsorbers ebenfalls verbessert wird.

Ein weiterer Aspekt der vorliegenden Erfindung beruht auf der Erkenntnis, dass die Verträglichkeit eines oral verabreichten Eisen-basierenden Phosphatadsorbers durch Coadministration mit einem physiologisch verträglichen Anionenaustauscher verbessert wird. Es wird angenommen, dass die Coadministration eines Anionenaustauschers eine durch den Phosphatadsorber verursachte verringerte Bindung von Gallensäuren im Gastrointestinaltrakt ausgleicht, wodurch die Verträglichkeit des Phosphatadsorbers ebenfalls verbessert wird.

Ein Gegenstand der Erfindung ist daher ein medizinisches Präparat zur oralen Verabreichung, umfassend
(i) einen Eisen-basierenden Phosphatadsorber und
(ii) ein physiologisch verträgliches Calciumsalz und/oder
(iii) einen physiologisch verträglichen Anionenaustauscher.

Das medizinische Präparat kann die Komponenten (i) und (ii) und/oder (iii) in einer einzigen Dosisform, z.B. als Mischung, oder als separate Dosisformen enthalten.

Vorzugsweise ist das Präparat weitgehend frei von Magnesiumsalzen, d.h. frei von Magnesiumsalzen in einer physiologisch wirksamen Dosis. So ist der Gehalt an Magnesiumsalzen in einer Tagesdosis des Präparats zur Verabreichung an einen menschlichen Patienten z.B. ≤ 1 mmol, ≤ 0,1 mmol oder ≤ 0,01 mmol. Die Abwesenheit von Magnesiumsalzen in einer physiologisch wirksamen Dosis ist insbesondere zweckmäßig im Falle einer Verabreichung des Präparats an Patienten mit Unverträglichkeit gegenüber Eisen-basierenden Phosphatadsorbern, insbesondere an Patienten mit Diarrhoe. Die Anwesenheit von Magnesiumsalzen in einer physiologisch wirksamen Dosis kann sogar zu einer Erhöhung der Unverträglichkeit führen.

Das Präparat kann in Form von festen Dosierungseinheiten, z.B. Pulvern, Tabletten, Kapseln, Sachets etc., gegebenenfalls überzogen mit einer Magensäure-resistenten Schicht, oder in Form von flüssigen Dosierungseinheiten, z.B. Ampullen, vorliegen. In einer bevorzugten Form liegt das Präparat als Pulver vor, das zur Verabreichung Nahrungsmitteln oder Getränken zugesetzt werden kann. Die Verabreichung der Komponenten (i) und (ii) und/oder (iii) kann gemeinsam oder separat, z.B. zu unterschiedlichen Zeiten, erfolgen.

Die Herstellung von Eisen-basierenden Phosphatadsorbern ist beispielsweise in DE 42 39 442 (SeBo GmbH) oder EP 0 868 125 (Vifor) beschrieben, auf deren Offenbarung hiermit Bezug genommen wird. Bevorzugt handelt es sich um Eisenhydroxid- und/oder Eisenoxidhydroxidenthaltende Phosphatadsorber, die zusätzlich mit Kohlenhydraten wie etwa Saccharose, Dextrinen versetzt und/oder komplexiert sein können. Vorzugsweise weist der Phosphatadsorber eine Phosphatbindungskapazität im Bereich von 0,05-2,5 mmol/g Trockengewicht, insbesondere von 0,5-2 mmol/g Trockengewicht auf. Besonders bevorzugt sind die Produkte SBR 759 (Novartis Pharma AG) oder PA 21 (Vifor Pharma-Vifor International lnc.).

Das Präparat enthält vorzugsweise eine Tagesdosis des Phosphatadsorbers oder einen Teil der Tagesdosis. Eine Tagesdosis des Phosphatadsorbers bei Verabreichung an einen menschlichen Patienten beträgt üblicherweise von 2-20 g, und insbesondere von 3-15 g oder 8-12 g Phosphatadsorber (jeweils Trockengewicht). Eine besonders bevorzugte Tagesdosis ist 10 g (Trockengewicht). Eine Tagesdosis kann in einer oder mehreren festen oder flüssigen Dosierungseinheiten (Tabletten, Kapseln, Sachets, Ampullen etc.) verabreicht werden.

Das physiologisch verträgliche Calciumsalz kann ein anorganisches oder organisches Calciumsalz sein und z.B. aus Calciumcarbonat, Calciumhydrogencarbonat, Calciumacetat, Calciumtartrat, Calciumgluconat oder Mischungen von einem oder mehreren dieser Salze ausgewählt sein. Das Präparat enthält vorzugsweise eine Tagesdosis des Calciumsalzes oder einen Teil der Tagesdosis. Eine Tagesdosis des Calciumsalzes bei Verabreichung an einen menschlichen Patienten beträgt üblicherweise 1-50 mmol, insbesondere von 5-25 mmol Calcium. Eine bevorzugte Tagesdosis enthält 20 mmol Calcium. Unter bestimmten Voraussetzungen kann auch zumindest zeitweise eine höhere Dosis des Calciumsalzes verabreicht werden. Eine Tagesdosis kann in einer oder mehreren festen oder flüssigen Dosierungseinheiten (z.B. Tabletten, Kapseln, Sachets, Ampullen etc.) gemeinsam mit dem Phosphatadsorber und gegebenenfalls dem Anionenaustauscher oder in separaten Dosierungseinheiten verabreicht werden.

Der physiologisch verträgliche Anionenaustauscher kann ein Anionenaustauscher auf Polymer/Copolymerbasis sein, der kationische organische Gruppen, z.B. quaternäre Ammoniumgruppen enthält. Beispiele für geeignete Anionenaustauscher sind Cholestyramin, Sevelamer, AMG 223 und MCI-196. Das Präparat enthält vorzugsweise eine Tagesdosis des Anionenaustauschers oder einen Teil der Tagesdosis. Eine Tagesdosis des Anionenaustauschers bei Verabreichung an einen menschlichen Patienten beträgt üblicherweise von 0,5-15 g und insbesondere von 5-10 g (jeweils Trockengewicht). Eine Tagesdosis kann in einer oder mehreren festen oder flüssigen Dosierungseinheiten (z,B. Tabletten, Kapseln, Sachets, Ampullen etc.) gemeinsam mit dem Phosphatadsorber und gegebenenfalls dem Calciumsalz oder in separaten Dosierungseinheiten verabreicht werden. In bestimmten Ausführungsformen der Erfindung beträgt das Gewichtsverhältnis von Eisen-basierendem Phosphatadsorber und Anionenaustauscher von 1:5 bis 20:1 und insbesondere von 1:1 bis 10:1.

Der Eisen-basierende Phosphatadsorber ist zur Behandlung der Hyperphosphatämie geeignet, die insbesondere bei Patienten mit fehlender oder eingeschränkter Nierenfunktion wie etwa Dialysepatienten auftritt. Das erfindungsgemäße Kombinationspräparat wird vorzugsweise zur Behandlung solcher Patienten eingesetzt, wobei eine Anwendung in der Human- und in der Veterinärmedizin vorgesehen ist. Die Anwendung in der Humanmedizin ist bevorzugt.

Insbesondere wird das erfindungsgemäße Präparat zur Behandlung von Personen mit Unverträglichkeit gegenüber einem Eisen-basierenden Phosphatadsorber eingesetzt. Diese Unverträglichkeit kann sich beispielsweise durch Auftreten von milder bzw. moderater Diarrhoe nach Einnahme des Phosphatadsorbers zeigen. Andererseits kann sich eine solche Unverträglichkeit auch durch eine unerwünschte Verringerung des Calciumspiegels im Serum, z.B. auf eine Calciumkonzentration von weniger als 2 mmol/l, zeigen. In einer Ausführungsform kann die Gabe des Calciumsalzes beim Auftreten derartiger Unverträglichkeiten nach Beginn einer Behandlung mit Phosphatadsorber verordnet werden. Alternativ kann die Gabe des Calciumsalzes gleich zu Beginn der Behandlung mit dem Phosphatadsorber verordnet werden. Somit kann das erfindungsgemäße Präparat sowohl zur Behandlung spezifischer Gruppen von Hyperphosphatämie-Patienten, z.B. von Patienten mit Unverträglichkeiten, aber auch zur Behandlung von Patienten ohne Unverträglichkeiten verwendet werden. Die Gabe des Calciumsalzes und/oder des Anionenaustauschers kann dauerhaft während der Behandlung mit dem Phosphatadsorber erfolgen. Andererseits ist auch eine intermittierende Gabe des Calciumsalzes und/oder des Anionenaustauschers möglich, d.h. die Behandlung umfasst Intervalle, in denen das Calciumsalz und/oder der Anionenaustauscher gemeinsam mit dem Phosphatadsorber, und Intervalle, in denen der Phosphatadsorber alleine verabreicht wird.

Ein weiterer Gegenstand der Erfindung ist die Verwendung eines oral verabreichten Eisen-basierenden Phosphatadsorbers in Kombination mit einer Calcium-Supplementation und/oder der Verabreichung eines physiologisch verträglichen Anionenaustauschers. Diese Verwendung erfolgt insbesondere für medizinische Zwecke in der Human- oder Veterinärmedizin. Bevorzugt erfolgt eine Behandlung der Hyperphosphatämie in Verbindung mit den bei Verabreichung eines Eisen-basierenden Phosphatadsorbers auftretenden Unverträglichkeiten. Vorzugsweise erfolgt die Verwendung in Abwesenheit einer physiologisch wirksamen Dosis von Magnesiumsalzen.

Die Calcium-Supplementation kann dabei, wie oben beschrieben, die Verabreichung eines physiologisch verträglichen Calciumsalzes umfassen. Es wird in diesem Zusammenhang auf die entsprechenden Ausführungen ausdrücklich Bezug genommen.

Alternativ oder zusätzlich kann eine Calcium-Supplementation auch durch diätetische Calciumzufuhr, d.h. durch Verabreichung calciumreicher Nahrungsmittel wie etwa Milchprodukte, z.B. Joghurt, Quark oder Hartkäse, sowie Tofu oder Fischprodukte, z.B. Sardinen, erfolgen. Bei einer derartigen diätetischen Calcium-Supplementation ist vorzugsweise die Zufuhr von 1-50 mmol, insbesondere 5-25 mmol Calcium täglich vorgesehen. Unter bestimmten Umständen kann zumindest zeitweise eine höhere Dosis von Calcium diätetisch zugeführt werden.

Die Verabreichung des physiologisch verträglichen Anionenaustauschers kann dabei wie oben beschrieben erfolgen. Es wird in diesem Zusammenhang auf die entsprechenden Ausführungen ausdrücklich Bezug genommen.

## Patentansprüche

1. Medizinisches Präparat zur oralen Verabreichung, umfassend
(i) einen Eisen-basierenden Phosphatadsorber und
(ii) ein physiologisch verträgliches Calciumsalz und/oder
(iii) einen physiologisch verträglichen Anionenaustauscher,
wobei das Präparat frei von einer physiologisch wirksamen Dosis von Magnesiumsalzen ist.

2. Präparat nach Anspruch 1,
**dadurch gekennzeichnet,**
**dass** es die Komponenten (i) und (ii) und/oder (iii) in einer einzigen Dosisform oder als separate Dosisformen enthält.

3. Präparat nach Anspruch 1 oder 2,
**dadurch gekennzeichnet,**
**dass** die Komponenten (i) und (ii) und/oder (iii) als Pulver vorliegen.

4. Präparat nach einem der Ansprüche 1 bis 3,
**dadurch gekennzeichnet,**
**dass** es eine Dosis der Komponente (i) von 3-15 g, insbesondere von 8-12 g Phosphatadsorber (jeweils Trockengewicht) enthält, und/oder dass es eine Dosis der Komponente (ii) von 1-30 mmol, insbesondere von 5-25 mmol Calcium enthält, und/oder dass es eine Dosis der Komponente (iii) von 0,5-15 g, insbesondere von 5-10 g Anionenaustauscher (jeweils Trockengewicht) enthält.

5. Präparat nach einem der Ansprüche 1 bis 4,
**dadurch gekennzeichnet,**
**dass** es einen Eisenhydroxid- und/oder Eisenoxidhydroxid-basierenden Phosphatadsorber, insbesondere das Produkt SBR 759 oder das Produkt PA 21 enthält.

6. Präparat nach einem der Ansprüche 1 bis 5,
**dadurch gekennzeichnet,**
**dass** das physiologisch verträgliche Calciumsalz ausgewählt ist aus Calciumcarbonat, Calciumhydrogencarbonat, Calciumacetat, Calciumtartrat, Calciumgluconat und Mischungen davon.

7. Präparat nach einem der Ansprüche 1 bis 6,
**dadurch gekennzeichnet,**
**dass** der physiologisch verträgliche Anionenaustauscher ein Anionenaustauscher auf Polymer/Copolymerbasis mit kationischen organischen Gruppen, insbesondere Cholestyramin oder Sevelamer ist.

8. Präparat nach einem der Ansprüche 1 bis 7 zur Behandlung der Hyperphosphatämie, z.B. in Patienten mit fehlender oder eingeschränkter Nierenfunktion, insbesondere zur Behandlung von Dialysepatienten.

9. Präparat nach einem der Ansprüche 1 bis 8 zur Behandlung von Personen mit Unverträglichkeit gegenüber einem Eisen-basierenden Phosphatadsorber, insbesondere zur Vermeidung des Auftretens von Diarrhoe.

10. Eisen-basierender Phosphatadsorber zur oralen Verabreichung in Kombination mit einer Calcium-Supplementation und/oder mit der Verabreichung eines physiologisch verträglichen Anionenaustauschers zur medizinischen Verwendung in Abwesenheit einer physiologisch wirksamen Dosis von Magnesiumsalzen.

11. Phosphatadsorber zur Verwendung nach Anspruch 10,
**dadurch gekennzeichnet,**
**dass** die Calcium-Supplementation die Verabreichung eines physiologisch verträglichen Calciumsalzes umfasst.

12. Phosphatadsorber zur Verwendung nach Anspruch 10,
**dadurch gekennzeichnet,**
**dass** die Calcium-Supplementation die Verabreichung calciumreicher Nahrungsmittel wie etwa Milchprodukte umfasst.

13. Phosphatadsorber zur Verwendung nach einem der Ansprüche 10 bis 12 zur Behandlung der Hyperphosphatämie, z.B. in Patienten mit fehlender oder eingeschränkter Nierenfunktion, insbesondere zur Behandlung von Dialysepatienten.

14. Phosphatadsorber zur Verwendung nach einem der Ansprüche 10 bis 13 zur Behandlung von Personen mit Unverträglichkeit gegenüber einem Eisen-basierenden Phosphatadsorber, insbesondere zur Vermeidung des Auftretens von Diarrhoe.

15. Phosphatadsorber zur Verwendung nach einem der Ansprüche 10 bis 14, der ein Eisenhydroxid- und/oder Eisenoxidhydroxid-basierender Phosphatadsorber, insbesondere das Produkt SBR 759 oder das Produkt PA 21 ist.
